# EUROPEAN PATENT APPLICATION

(11) **EP 4 327 951 A1**
(43) Date of publication of application: **28.02.2024**
(21) Application number: 21932415.9
(22) Date of filing: 04.06.2021
(51) Int. Cl.: B05B 11/06

(54) **STATIC ELECTRICITY GENERATING APPARATUS OF ATOMIZER**

(30) Priority: 26.03.2021 CN 202120625236 U
(71) Applicant: Zhejiang Prulde Electric Appliance Co., Ltd., Jinhua, Zhejiang 321035 (CN)
(72) Inventor: YANG, Weiming, Jinhua, Zhejiang 321035 (CN)
(74) Representative: Ipside
(86) International application number: PCT/CN2021/098435
(87) International publication number: WO 2022/198799

(57) **Abstract**

This invention provides an electrostatic generation device for an atomizer. It solves the problem of poor spraying effect of the atomizer. When this utility model is used, the fan blows air through the water outlet pipe, and part of the airflow passes through the pressure tube into the water tank, continuously increasing the air pressure inside the water tank. The greater pressure forces the liquid inside the water tank into the inlet pipe and along the inlet pipe into the guide tube, spraying out from the guide tube in the direction away from the fan through the water outlet pipe. The liquid is electrified by the charged metal valve core, and the electrostatically charged liquid is sprayed out through the air outlet, forming electrostatically charged atomized particles. The spray can easily adhere to the object being sprayed, resulting in a higher spraying efficiency.

## Description

### Technical Field

This invention relates to the technical field of electric tools, and particularly to an electrostatic generation device for an atomizer.

### Background Art

Atomizers are commonly used to spray medicinal solutions or solvents in the form of mist and vapor. Typically, atomizers operate by rotating an internal fan, which blows air through the air outlet pipe, creating an airflow from inside to outside. Then, by pressurizing the interior of the water tank with an air pump, the liquid inside the water tank is forced under pressure along the connecting pipeline between the water outlet pipe and the water tank into the air outlet pipe. The liquid entering the air outlet pipe is blown outwards by the fan in the form of mist and vapor. However, the liquid inside the water tank is sprayed out after passing through a guide tube, in which a movable plug is set to adjust the gap size between the plug and the air outlet of the guide tube. The water and gas inside the atomizer are sprayed out through this gap. This spraying method produces uneven spray that does not adhere well to the sprayed objects, resulting in less-than-ideal spray effects. Moreover, it consumes a large amount of water and medicinal solution, leading to low spraying efficiency. There is room for improvement in the existing technology.

### Summary of the Invention

This invention aims to provide an electrostatic generation device for an atomizer with better spraying effects, addressing the shortcomings of poor spraying effects of conventional atomizers.

The purpose of this invention is achieved through the following technical solutions: an electrostatic spraying device for an atomizer, comprising a housing, a water outlet pipe, a fan that blows air through the water outlet pipe, a motor that drives the fan, a water tank, and a power supply that powers the motor. It is characterized by a guide tube fixed inside the water outlet pipe that directs water away from the fan. The end of the guide tube away from the fan has an air outlet, and inside the guide tube, there is a movable conductive valve core that can move along the length of the guide tube and adjust the size of the air outlet. The end of the conductive valve core away from the air outlet of the guide tube is connected to a high voltage lead that is electrically connected to the power supply.

Preferably, the end of the conductive valve core away from the air outlet of the guide tube is equipped with a second spring that drives the conductive valve core to move towards the air outlet of the guide tube.

Preferably, the water outlet pipe has a pressure tube connected to the water tank, and the guide tube has an inlet pipe connected to the water tank. The end of the inlet pipe away from the guide tube is located at the bottom of the water tank. The housing is equipped with a control switch for controlling the opening and closing of the fan and the water outlet pipe. The control switch includes a push button for controlling the fan and a button for driving the adjusting mechanism to move. The housing has a sliding slot for engaging the push button and allowing it to reciprocate along its length. A rotating rod is fixed inside the housing, and the button is rotatably mounted on this rotating rod. One end of the button is located below the push button, and the other end abuts the adjusting mechanism. The lower end of the push button is fixed with a fixed rod. The upper end surface of the button, away from the adjusting mechanism, has a notch for engaging the fixed rod. The push rod can move towards the button along the sliding slot and by pressing the button, it rotates around the rotating rod, causing the notch on the button to rotate upwards and engage the fixed rod.

Preferably, the end of the fixed rod facing the button is fixed with a fixed column, around which a first spring is fitted that drives the push button to move away from the button. The housing is fixed with a snap seat for engaging the first spring, with the opening facing the fixed rod. The end of the first spring away from the fixed column is engaged within the snap seat.

Preferably, a torsion spring is fitted on the rotating rod, which drives the end of the button away from the adjusting mechanism to rotate downwards around the rotating rod. A snap post is fixed on the housing above the button, and one end of the torsion spring is engaged on this snap post, with the other end fixed on the button.

Preferably, the upper end of the push button is fixed with a push block, the upper end of which is located outside the housing. The housing has an active slot for engaging the push block, with the length of the active slot being greater than the length of the push block. The push button is marked with on and off labels, with the two labels located on either side of the push block on the push button, in line with the push block and parallel to the length direction of the push button. This allows for a more intuitive display of the working status of the atomizer.

Preferably, the control switch is equipped with an adjusting mechanism for adjusting the water output size of the water outlet pipe. The adjusting mechanism includes a splined shaft that rotates within the housing and a rotating sleeve that is fitted over the splined shaft and can move along the length of the splined shaft. The inner wall of the rotating sleeve has a guide groove that matches the splined shaft. The housing is fixed with a limit plate that restricts the movement of the rotating sleeve along the length of the splined shaft. The lower end surface of the splined shaft has a rotation groove that extends into the interior of the splined shaft. A push rod is movably set within the rotation groove and is threadedly connected within the rotation groove. The push rod has a guide plate fixed on either side, and the housing is fixed with a snap groove for the push rod, allowing the guide plate to move along the length of the push rod within the snap groove. The housing is equipped with an adjusting knob for controlling the rotation of the rotating sleeve.

Preferably, the adjusting knob is rotatably mounted on the housing, and the end of the rotating sleeve facing the push rod is fixed with a first gear. The end of the adjusting knob facing the rotating sleeve is fixed with a second gear that always meshes with the first gear. The adjusting knob can drive the rotating sleeve to rotate around its central axis through the meshing of the first and second gears.

Preferably, the first gear is a helical gear, and the second gear gradually inclines towards the periphery of the adjusting knob from the direction of the rotating sleeve. The length direction of the teeth on the second gear is perpendicular to the length direction of the teeth on the first gear.

Preferably, the end of the rotating sleeve facing the push rod is fixed with a limit ring, which is located inside the first gear and fitted over the push rod. The inner diameter of the limit ring is smaller than the outer diameter of the splined shaft. A positioning ring is fixed on the push rod, with a diameter larger than that of the push rod but smaller than the inner diameter of the limit ring.

Preferably, the housing is equipped with a grounding wire, which is positioned so that when the user grips the housing, the grounding wire touches the user's hand.

An electrostatic generation device for an atomizer, comprising a housing, a water outlet pipe, a fan that blows air through the water outlet pipe, a motor that drives the fan, a water tank, an electrostatic generator, and a power supply that powers the motor. It is characterized by a guide tube fixed inside the water outlet pipe that directs water away from the fan. The end of the guide tube away from the fan has an air outlet and a water outlet; one pole of the electrostatic generator contacts the liquid to charge it, and the method of contact with the liquid is at least one of the following:
(1) One pole of the electrostatic generator is connected inside the water tank, charging the liquid upon contact;
(2) One pole of the electrostatic generator is connected at the water outlet, charging the liquid upon contact;
(3) One pole of the electrostatic generator is connected inside the pipeline between the water tank and the water outlet, charging the liquid upon contact.

An electrostatic generation device for an atomizer, comprising a housing, a water outlet pipe, a fan that blows air through the water outlet pipe, a motor that drives the fan, a water tank, an electrostatic generator, and a power supply that powers the motor. It is characterized by a guide tube fixed inside the water outlet pipe that directs water away from the fan. The end of the guide tube away from the fan has an air outlet and a water outlet; the first electrode of the electrostatic generator contacts the liquid, while the second electrode does not contact the liquid. The connection location of the second electrode is at least one of the following:
(1) The second pole of the electrostatic generator is connected at the handle, in contact with the user;
(2) The second pole of the electrostatic generator is connected on a sharp needle in front of the water outlet;
(3) The second pole of the electrostatic generator is connected on an electrostatic ring outside the water outlet.

Compared with existing technology, this invention uses a fan to blow air through the water outlet pipe. Part of the airflow enters the water tank through the pressure tube, continuously increasing the air pressure inside the water tank. The increased pressure forces the liquid inside the water tank into the inlet pipe and then into the guide tube, spraying out from the guide tube away from the fan. The liquid is charged by the conductive valve core carrying an electric charge, and the charged liquid is sprayed out through the air outlet, forming electrostatically charged mist particles. The spray can easily adhere to the sprayed objects, resulting in higher spraying efficiency.

### Brief Description of the Drawings

Figure 1 is an overall structural view of this invention;
Figure 2 is an internal structural view of the atomizer;
Figure 3 is a sectional view of the internal structure of the atomizer;
Figure 4 is an enlarged view of section D in Figure 3;
Figure 5 is an enlarged view of section C in Figure 2;
Figure 6 is an overall structural view of the control switch and the adjusting mechanism;
Figure 7 is a sectional view of the control switch;
Figure 8 is an overall structural view of the control switch in the closed state;
Figure 9 is an enlarged view of section A in Figure 2;
Figure 10 is an overall structural view of the adjusting mechanism;
Figure 11 is an exploded structural view of the adjusting mechanism;
Figure 12 is a sectional view of the adjusting mechanism;
Figure 13 is an enlarged view of section B in Figure 12;
Figure 14 is a sectional view of the water outlet pipe area.

List of reference numerals:
1 - housing; 2 - button; 3 - water outlet pipe; 4 - splined shaft; 5 - rotating sleeve; 6 - guide groove; 7 - limit plate; 8 - rotation groove; 9 - push rod; 10 - guide plate; 11 - snap groove; 12 - adjusting knob; 13 - first gear; 14 - second gear; 15 - limit ring; 16 - positioning ring; 17 - pressing block; 18 - control switch; 19 - adjusting mechanism; 20 - push button; 21 - sliding slot; 22 - rotating rod; 23 - fixed rod; 24 - notch; 25 - fixed column; 26 - first spring; 27 - snap seat; 28 - torsion spring; 29 - snap post; 30 - push block; 31 - active slot; 32 - label; 33 - water tank; 34 - fan; 35 - battery compartment; 36 - cover plate; 37 - guide tube; 38 - conductive valve core; 39 - plug; 40 - toggle block; 41 - pressure tube; 42 - inlet pipe; 43 - sealing ring; 44 - flange; 45 - motor; 46 - power supply; 47 - high voltage lead; 48 - second spring; 49 - air outlet; 50 - grounding wire; 51 - electrostatic generator; 52 - water outlet.

### Detailed Description of Embodiments

This invention is further described below with reference to the embodiments shown in the drawings.

### Embodiment 1:

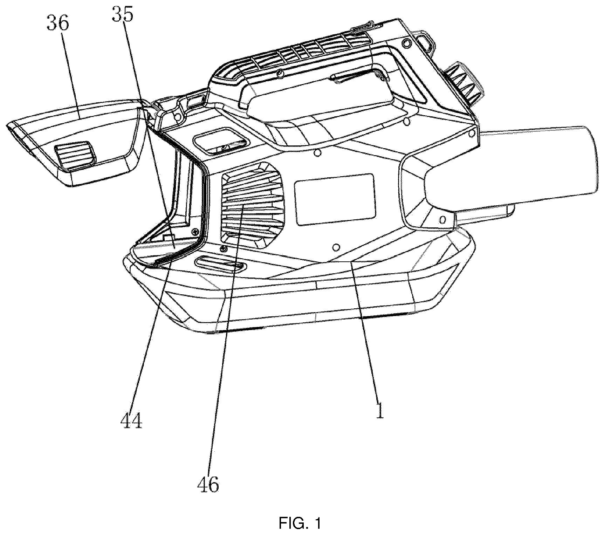

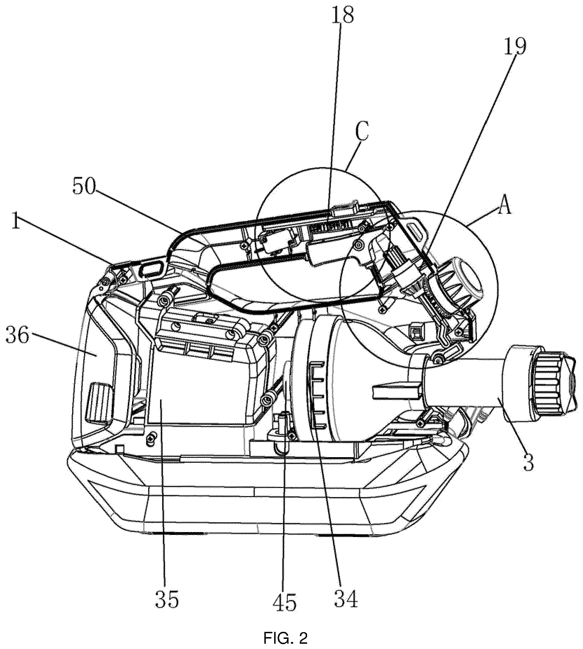

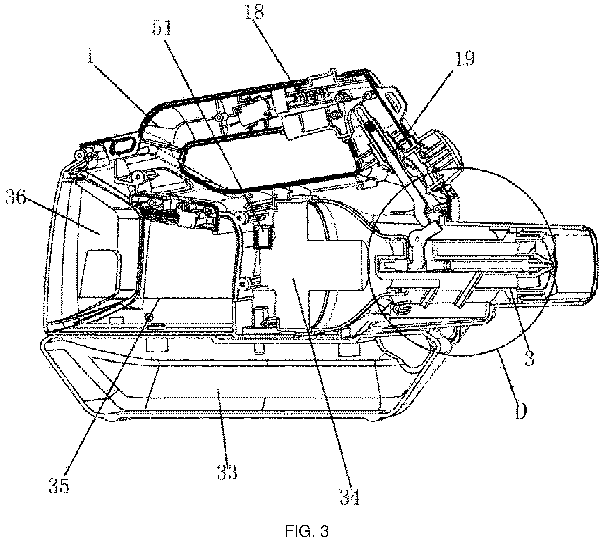

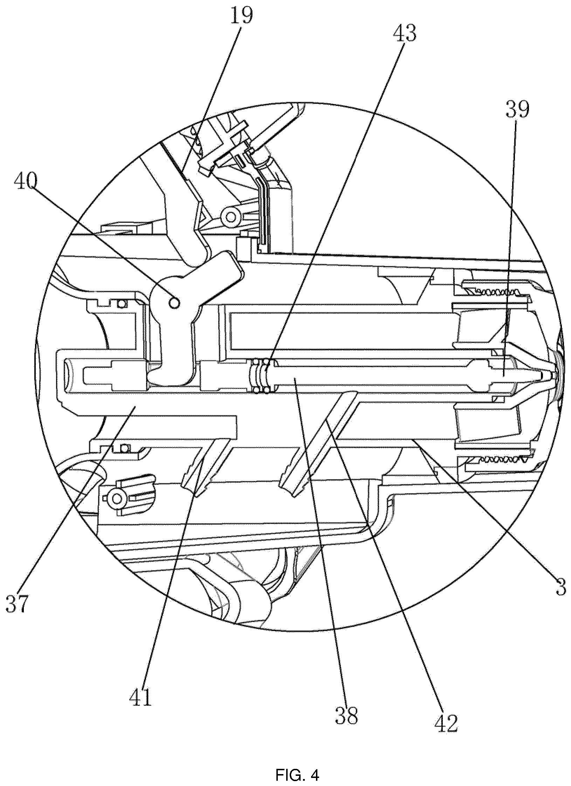

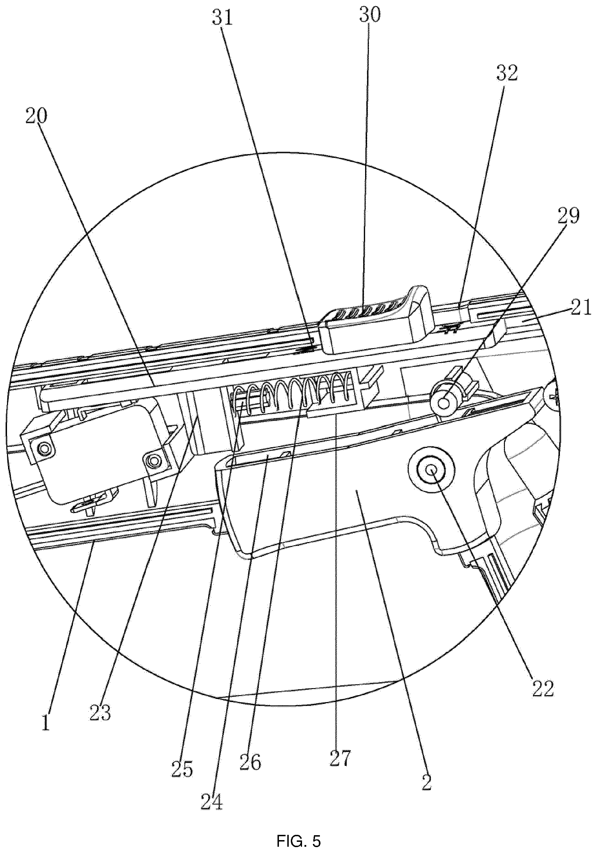

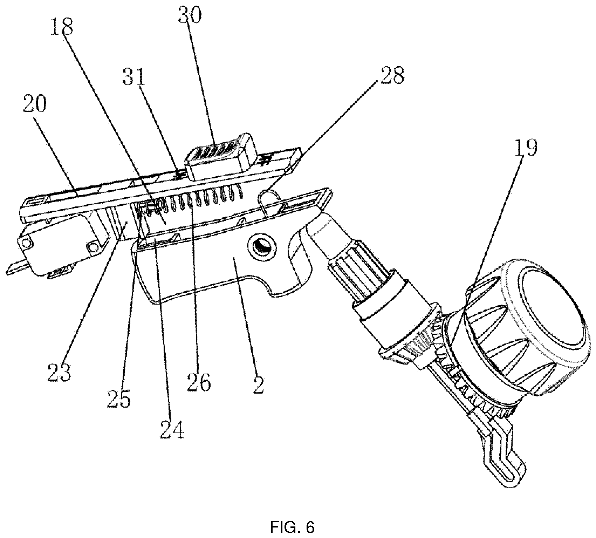

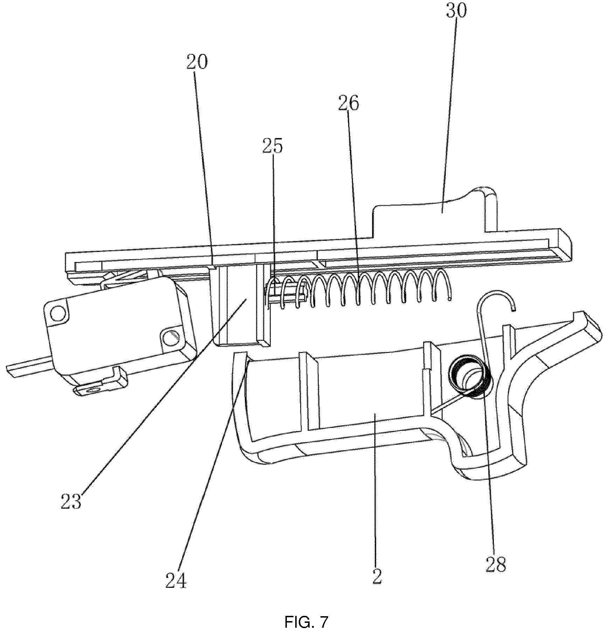

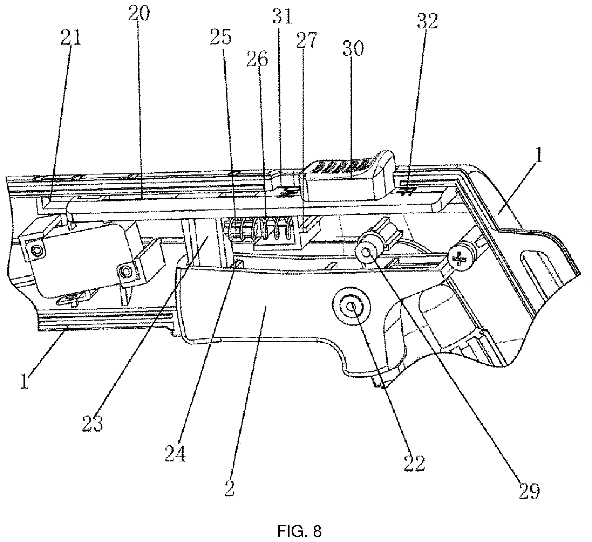

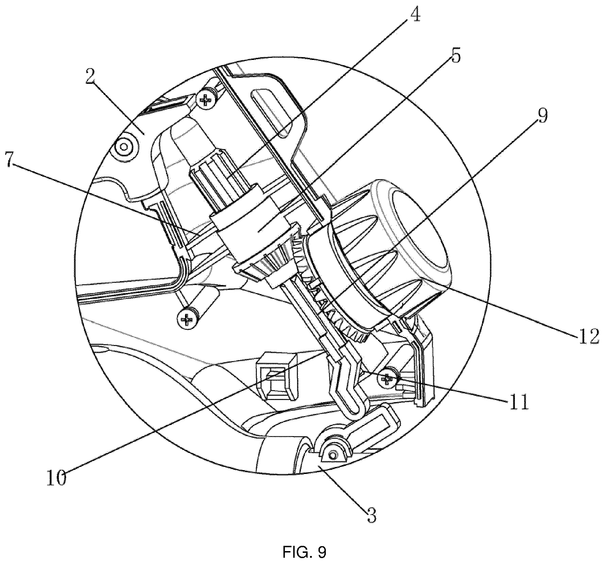

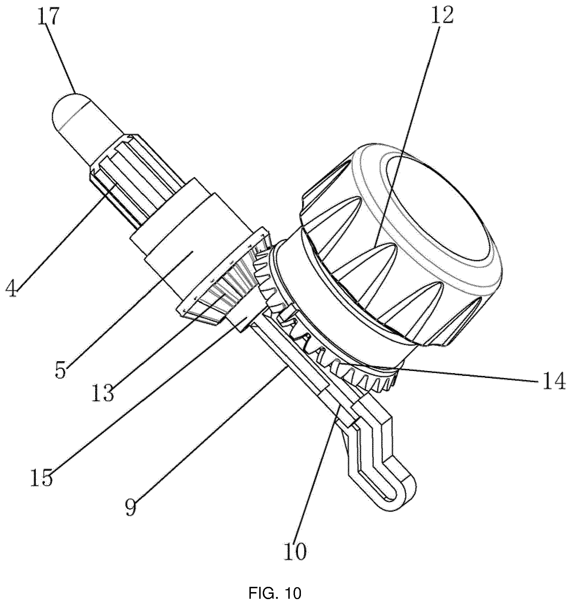

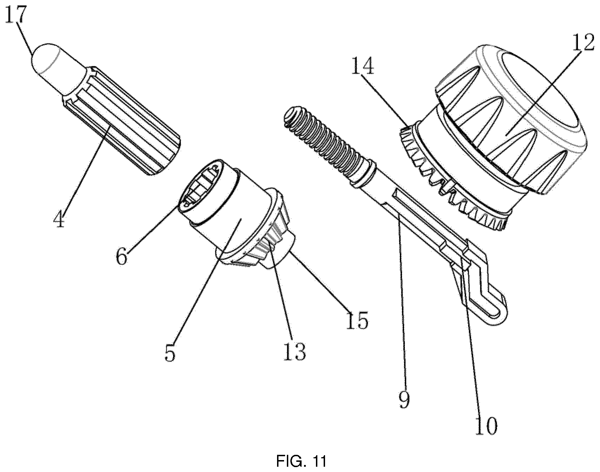

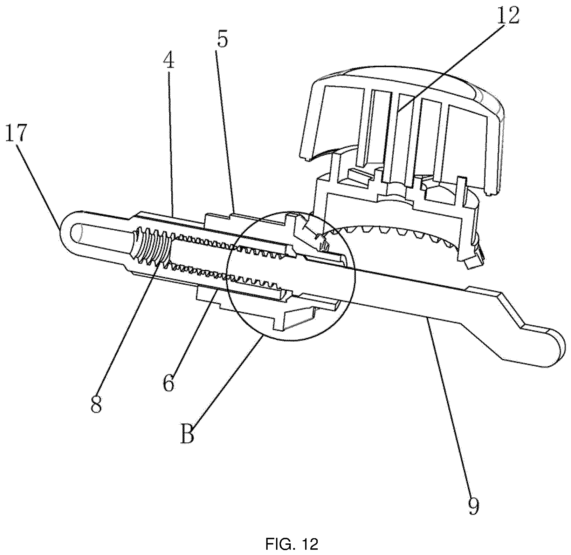

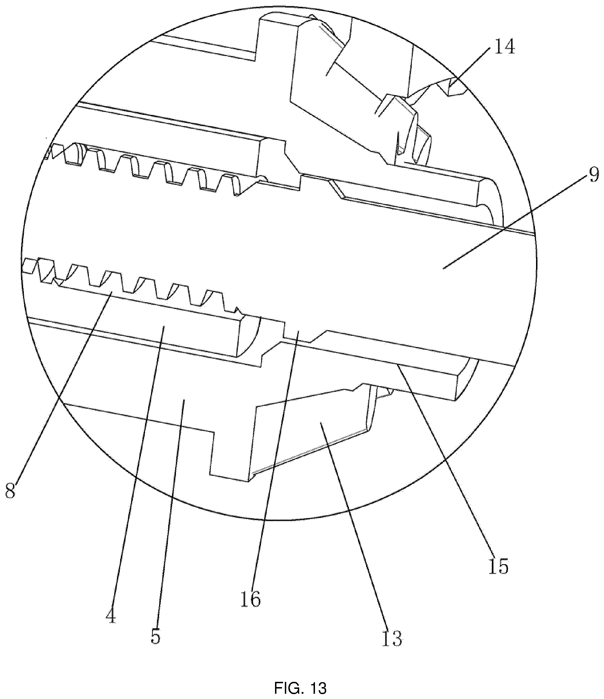

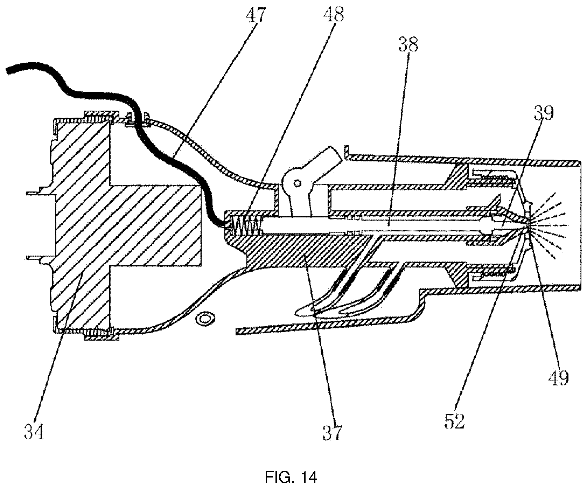

As shown in Figures 1-14, an electrostatic generation device for an atomizer includes a housing 1 and a water tank 33 fixed at the lower end of the housing. The housing 1 is fixed with a water outlet pipe 3, a fan 34 that blows air through the water outlet pipe 3, a motor 45 that drives the fan 34, and a power supply 46 that powers the motor 45. The power supply 46 includes a battery compartment 35 located at the end of the housing 1 away from the water outlet pipe 3 for installing a removable battery pack to power the motor. The housing 1 is rotatably fitted with a cover plate 36 that can be snapped onto the battery compartment 35. The housing 1 is equipped with a control switch 18 for controlling the opening and closing of the fan 34 and the water outlet pipe 3. The control switch 18 is equipped with an adjusting mechanism 19 for adjusting the water output size of the water outlet pipe 3. The control switch 18 includes a push button 20 for controlling the fan controller and a button 2 for driving the adjusting mechanism 19 to move. The housing 1 has a sliding slot 21 for engaging the push button 20, allowing it to reciprocate along its length. A rotating rod22 is fixed inside the housing 1, and the button 2 is rotatably mounted on this rotating rod 22. One end of the button 2 is located below the push button 20, and the other end abuts the adjusting mechanism 19. The lower end of the push button 20 is fixed with a fixed rod 23. The upper end surface of the button 2, away from the adjusting mechanism 19, has a notch 24 for engaging the fixed rod 23. The push rod can move towards the button 2 along the sliding slot 21 and by pressing the button 2, it rotates around the rotating rod 22, causing the notch 24 on the button to rotate upwards and engage the fixed rod 23. The housing is equipped with a grounding wire, which is positioned so that when the user grips the housing, the grounding wire touches the user's hand, grounding the static electricity from the user through the grounding wire to ensure the normal operation of the device.

The housing 1 has a flange 44 for engaging the cover plate 36, which snaps onto the flange 44 after being closed, effectively protecting the removable battery located inside the battery compartment 35.

The end of the fixed rod 23 facing the button 2 is fixed with a fixed column 25, around which a first spring 26 is fitted that drives the push button 20 to move away from the button 2. The housing 1 is fixed with a snap seat 27 for engaging the first spring 26, with the opening facing the fixed rod 23. The end of the first spring 26 away from the fixed column 25 is engaged within the snap seat 27. A torsion spring 28 is fitted on the rotating rod 22, which drives the end of the button 2 away from the adjusting mechanism 19 to rotate downwards around the rotating rod 22. A snap post 29 is fixed on the housing 1 above the button 2, and one end of the torsion spring 28 is engaged on this snap post 29, with the other end fixed on the button 2.

The upper end of the push button 20 is fixed with a push block 30, the upper end of which is located outside the housing 1. The housing 1 has an active slot 31 for engaging the push block 30, with the length of the active slot 31 being greater than the length of the push block 30. The push button 20 is marked with on and off labels 32, with the two labels 32 located on either side of the push block 30 on the push button 20, in line with the push block 30 and parallel to the length direction of the push button 20. This allows for a more intuitive display of the working status of the atomizer.

The water outlet pipe 3 is fitted over the fan 34, and the water outlet pipe 3 is fixed with a guide tube 37. The end of the guide tube 37 away from the fan 34 has an air outlet 49. Inside the guide tube 37, there is a movable conductive valve core 38 that can move along the length of the guide tube 37 to adjust the size of the air outlet. The conductive valve core 38 away from the guide tube 37 outlet is equipped with a second spring 48 that drives the conductive valve core 38 to move towards the air outlet 49 of the guide tube 37. The end of the conductive valve core 38 facing the opening of the guide tube 37 is fixed with a plug 39. The water outlet pipe 3 is rotatably fitted with a toggle block 40 for moving the conductive valve core 38 within the guide tube 37 along its length. The toggle block 40 abuts the lower end of the adjusting mechanism. The water outlet pipe 3 has a pressure tube 41 connected to the water tank, and the guide tube 37 has an inlet pipe 42 connected to the water tank. The end of the pressure tube 41 away from the water outlet pipe 3 is located at the upper end of the water tank, and the end of the inlet pipe 42 away from the guide tube 37 is located at the bottom of the water tank. Two sealing rings 43 are fixed around the conductive valve core 38, located between the toggle block 40 and the plug 39, preventing the liquid entering the guide tube 37 from flowing into the end of the guide tube 37 near the fan 34. The connection between the inlet pipe 42 and the guide tube 37 is located between the sealing rings 43 and the plug 39. By pressing the button 2, the adjusting mechanism 19 pushes the toggle block 40, driving the conductive valve core 38 to move along the guide tube 37 towards the fan, separating the plug 39 from the opening of the guide tube 37. Gas enters the water tank through the pressure tube 41, increasing the pressure above the liquid level in the water tank 33. Under the pressure, the liquid enters the guide tube 37 through the inlet pipe 42 and is sprayed out through the opening of the guide tube 37. By changing the length of the adjusting mechanism 19, the movement length of the conductive valve core 38 is adjusted, achieving the purpose of adjusting the gap size between the plug 39 of the conductive valve core 38 and the opening of the water outlet pipe 3. This adjusts the water output size, with a simple structure and convenient use. The second spring 48 resets the conductive valve core 38.

The liquid is charged by the conductive valve core 38 carrying an electric charge, and the charged liquid is sprayed out through the air outlet 49, forming electrostatically charged mist particles. The spray can easily adhere to the sprayed objects, resulting in higher spraying efficiency.

When in use, the push button 20 is moved towards the button 2 to start the fan controller, and the fixed rod 23 on the push button 20 is positioned above the notch 24 on the button 2. Squeezing the button 2 drives the end of the button 2 away from the adjusting mechanism 19 to rotate upwards around the rotating rod 22 until the notch 24 on the button 2 engages the lower end of the fixed rod 23, at which point the atomizer begins to output water. When either the push button 20 or the button 2 is pressed alone, no water is dispensed. The first spring 26 provides a force in the direction away from the button 2, ensuring that the lower end of the fixed rod 23 is securely engaged in the notch 24. The torsion spring 28 provides a force to move the end of the button 2 away from the adjusting mechanism 19 and rotate downwards around the rotating rod 22. When the user releases the button 2, under the action of the torsion spring 28, the end of the button 2 away from the adjusting mechanism 19 rotates downwards around the rotating rod 22, disengaging the notch 24 from the fixed rod 23. The fixed rod 23 moves in the direction away from the button 2 under the action of the first spring 26 along the sliding slot 21, controlling the fan controller to close. This is convenient for use and effectively prevents accidental activation of the atomizer due to inadvertent pressing of the button 2.

The adjusting mechanism 19 includes a splined shaft 4 that rotates within the housing 1 and a rotating sleeve 5 that is fitted over the splined shaft 4 and can move along the length of the splined shaft 4. The inner wall of the rotating sleeve 5 has a guide groove 6 that matches the splined shaft 4. The housing 1 is fixed with a limit plate 7 that restricts the movement of the rotating sleeve 5 along the length of the splined shaft 4. The lower end surface of the splined shaft 4 has a rotation groove 8, within which a push rod 9 is movably set and is threadedly connected within the rotation groove 8. The push rod 9 has a guide plate 10 fixed on either side, and the housing 1 is fixed with a snap groove 11 for the push rod, allowing the guide plate 10 to move along the length of the push rod 9 within the snap groove 11. The housing 1 is equipped with an adjusting knob 12 for controlling the rotation of the rotating sleeve 5.

The adjusting knob 12 is rotatably mounted on the housing 1, and the end of the rotating sleeve 5 facing the push rod 9 is fixed with a first gear 13. The end of the adjusting knob 12 facing the rotating sleeve 5 is fixed with a second gear 14 that always meshes with the first gear 13. The adjusting knob 12 can drive the rotating sleeve 5 to rotate around its central axis through the meshing of the first and second gears. By rotating the knob, the knob drives the rotating sleeve 5, which is fitted over the splined shaft 4, to rotate around the central axis of the splined shaft 4. The push rod 9 is restricted from rotating by the guide plates 10 fixed on either side of the push rod 9 and engaged in the snap groove 11. The splined shaft 4 and the push rod 9, which are threadedly connected, move the push rod 9 along the length of the snap groove 11 under the drive of the rotating splined shaft 4, achieving the purpose of adjusting the overall length of the splined shaft 4 and the push rod 9. By pressing the button 2, the adjusting mechanism 19 of different lengths is used to adjust the gap size between the plug in the water outlet pipe 3 and the air outlet 49, achieving the purpose of adjusting the water output size. The structure is simple and the adjustment is convenient.

The first gear 13 is a helical gear, and the second gear 14 gradually inclines towards the periphery of the adjusting knob 12 from the direction of the rotating sleeve 5. The length direction of the teeth on the second gear 14 is perpendicular to the length direction of the teeth on the first gear 13. The force of rotating the adjusting knob 12 is fully transferred to the first gear 13, making the rotation more effortless.

The end of the rotating sleeve 5 facing the push rod 9 is fixed with a limit ring 15, which is located inside the first gear 13 and fitted over the push rod 9. The inner diameter of the limit ring 15 is smaller than the outer diameter of the splined shaft 4. A positioning ring 16 is fixed on the push rod 9, with a diameter larger than that of the push rod 9 but smaller than the inner diameter of the limit ring 15. During assembly, the lower end of the splined shaft 4 is placed against the end of the limit ring 15 facing the splined shaft 4, and the push rod 9 is threadedly fitted into the splined shaft 4 until the positioning ring 16 abuts the end of the splined shaft 4 facing the push rod 9, completing the assembly. The positioning ring 16 defines the assembly position of the push rod 9, ensuring precise positioning during installation.

The end of the splined shaft 4 away from the push rod 9 is fixed with a pressing block 17. The pressing block 17 is spherical, and when the button 2 on the atomizer is pressed to drive the splined shaft 4 to move, the button 2 always abuts the pressing block 17. The button 2 remains in a rotating state during the pressing process, with its end surface always abutting the pressing block 17, avoiding any jerky sensation during pressing and making the pressing process smoother.

### Embodiment 2:

An electrostatic generation device for an atomizer, comprising a housing 1, a water outlet pipe 3, a fan 34 that blows air through the water outlet pipe 3, a motor 45 that drives the fan 34, a water tank 33, an electrostatic generator 51, and a power supply 46 that powers the motor 45. The water outlet pipe 3 is fixed with a guide tube 37 that directs water away from the fan 34. The end of the guide tube 37 away from the fan 34 has an air outlet 49 and a water outlet 52. One pole of the electrostatic generator 51 contacts the liquid to charge it, and the method of contact with the liquid is at least one of the following:
(1) One pole of the electrostatic generator is connected inside the water tank, charging the liquid upon contact;
(2) One pole of the electrostatic generator is connected at the water outlet, charging the liquid upon contact;
(3) One pole of the electrostatic generator is connected inside the pipeline between the water tank and the water outlet, charging the liquid upon contact.

### Embodiment 3:

An electrostatic generation device for an atomizer, comprising a housing 1, a water outlet pipe 3, a fan 34 that blows air through the water outlet pipe 3, a motor 45 that drives the fan 34, a water tank 33, an electrostatic generator 51, and a power supply 46 that powers the motor 45. It is characterized by a guide tube 37 fixed inside the water outlet pipe 3 that directs water away from the fan 34. The end of the guide tube 37 away from the fan 34 has an air outlet 49 and a water outlet 52. The first electrode of the electrostatic generator 51 contacts the liquid, while the second electrode does not contact the liquid. The connection location of the second electrode is at least one of the following:
(1) The second pole of the electrostatic generator is connected at the handle, in contact with the user;
(2) The second pole of the electrostatic generator is connected on a sharp needle in front of the water outlet;
(3) The second pole of the electrostatic generator is connected on an electrostatic ring outside the water outlet.

The specific embodiments described in this document are merely illustrative examples of the spirit of the invention. Those skilled in the art of this invention can make various modifications or additions to the described specific embodiments or use similar methods instead, without deviating from the spirit of the invention or exceeding the scope defined by the claims attached hereto.

## Claims

1. An electrostatic generation device for an atomizer, comprising a housing (1), a water outlet pipe (3), a fan (34) that blows air towards the water outlet pipe (3), a motor (45) that drives the fan (34) to rotate, a water tank (33), and a power supply (46) that supplies power to the motor (45), **characterized in that**, a guide tube (37) is fixedly installed inside the water outlet pipe (3) for directing water away from the fan (34), the end of the guide tube (37) away from the fan (34) is provided with an air outlet (49), and a conductive valve core (38) is movably set inside the guide tube (37) that can move along the length of the guide tube (37) and adjust the size of the air outlet (49), the end of the conductive valve core (38) away from the air outlet (49) of the guide tube (37) is provided with a high voltage lead (47) that is electrically connected to the power supply (46).

2. The electrostatic generation device for an atomizer according to claim 1, **characterized in that**, the end of the conductive valve core away from the outlet of the guide tube (37) is provided with a second spring that drives the conductive valve core to move towards the direction of the air outlet of the guide tube (37).

3. The electrostatic generation device for an atomizer according to claim 1, **characterized in that**, the water outlet pipe (3) is provided with a pressure tube (41) that connects to the water tank (33), the guide tube (37) is provided with an inlet pipe (42) that connects to the water tank (33), the end of the inlet pipe (42) away from the guide tube (37) is located at the bottom inside the water tank (33), the housing (1) is provided with a control switch (18) for controlling the opening and closing of the fan (34) and the water outlet pipe (3), the control switch (18) includes a push button (20) for controlling the opening and closing of the fan (34) and a button (2) for driving the adjusting mechanism (19) to move, the housing (1) is provided with a sliding slot (21) for engaging the push button (20) and allowing the push button (20) to reciprocate along its length direction, a rotating rod (22) is fixedly installed inside the housing (1), the button (2) is rotatably mounted on the rotating rod (22), one end of the button (2) is located below the push button (20), and the other end abuts the adjusting mechanism (19), the lower end of the push button (20) is fixedly provided with a fixed rod (23), the upper end surface of the button (2) away from the adjusting mechanism (19) is provided with a notch (24) for engaging the fixed rod (23), the push rod can move along the length direction of the sliding slot (21) towards the direction of the button (2) and by pressing the button (2) cause the button (2) to rotate around the rotating rod (22) so that the notch (24) on the button (2) rotates upwards around the rotating rod (22) and engages on the fixed rod (23).

4. The electrostatic generation device for an atomizer according to claim 3, **characterized in that**, the end of the fixed rod (23) facing the button (2) is fixedly provided with a fixed column (25), the outer circumference of the fixed column (25) is provided with a first spring (26) that drives the push button (20) to move away from the button (2), the housing (1) is fixedly provided with a snap seat (27) for engaging the first spring (26) with its opening facing the fixed rod (23), and the end of the first spring (26) away from the fixed column (25) is engaged inside the snap seat (27).

5. The electrostatic generation device for an atomizer according to claim 3, **characterized in that**, a torsion spring (28) is provided on the rotating rod (22) that drives the end of the button (2) away from the adjusting mechanism (19) to rotate downwards around the rotating rod (22), the housing (1) is fixedly provided with a snap post (29) located above the button (2), one end of the torsion spring (28) is engaged on the snap post (29), and the other end of the torsion spring (28) is fixedly installed on the button (2).

6. The electrostatic generation device for an atomizer according to claim 5, **characterized in that**, the upper end of the push button (20) is fixedly provided with a push block (30), the upper end of the push block (30) is located outside the housing (1), the housing (1) is provided with an active slot (31) for engaging the push block (30), the length of the active slot (31) is greater than the length of the push block (30), the push button (20) is provided with labels (32) for on and off, the two labels (32) are located on both sides of the push block (30) on the push button (20), and the two labels (32) and the push block (30) are on the same line and parallel to the length direction of the push button (20).

7. The electrostatic generation device for an atomizer according to claim 1, **characterized in that**, the control switch (18) is provided between the water outlet pipe (3) for adjusting the water output size of the water outlet pipe (3), the adjusting mechanism (19) includes a splined shaft (4) that is rotatably mounted inside the housing (1) and a rotating sleeve (5) that is fitted over the splined shaft (4) and can move along the length of the splined shaft (4), the inner wall of the rotating sleeve (5) is provided with a guide groove (6) that matches the splined shaft (4), the housing (1) is fixedly provided with a limit plate (7) for restricting the movement of the rotating sleeve (5) along the length of the splined shaft (4), the lower end surface of the splined shaft (4) is provided with a rotation groove (8) that extends into the interior of the splined shaft (4), a push rod (9) is movably set inside the rotation groove (8) and is movably mounted inside the rotation groove (8) through a threaded connection, the two sides of the push rod (9) are fixedly provided with a guide tube (37), the housing (1) is fixedly provided with a snap groove (11) for the push rod and allows the guide tube (37) (10) to move along the length of the push rod (9) inside the snap groove (11), the housing (1) is provided with an adjusting knob (12) for controlling the rotation of the rotating sleeve (5).

8. The electrostatic generation device for an atomizer according to claim 7, **characterized in that**, the adjusting knob (12) is rotatably mounted on the housing (1), the end of the rotating sleeve (5) facing the push rod (9) is fixedly provided with a first gear (13), the end of the adjusting knob (12) facing the rotating sleeve (5) is fixedly provided with a second gear (14) that is always engaged with the first gear (13), the adjusting knob (12) can drive the rotating sleeve (5) to rotate around the central axis of the rotating sleeve (5) through the mutually cooperating first gear (13) and second gear (14).

9. The electrostatic generation device for an atomizer according to claim 8, **characterized in that**, the first gear (13) is a helical gear, the second gear (14) gradually inclines towards the periphery of the adjusting knob (12) from the adjusting knob (12) towards the rotating sleeve (5), and the length direction of the teeth on the second gear (14) is perpendicular to the length direction of the teeth on the first gear (13).

10. The electrostatic generation device for an atomizer according to claim 9, **characterized in that**, the end of the rotating sleeve (5) facing the push rod (9) is fixedly provided with a limit ring (15), the limit ring (15) is located inside the first gear (13) and fitted over the outer circumference of the push rod (9), the inner diameter of the limit ring (15) is smaller than the outer diameter of the splined shaft (4), the push rod (9) is fixedly provided with a positioning ring (16), the diameter of the positioning ring (16) is larger than the diameter of the push rod (9) and smaller than the inner diameter of the limit ring (15).

11. The electrostatic generation device for an atomizer according to claim 1, the housing (1) is provided with a grounding wire (50), the grounding wire is positioned so that when the user grasps the housing, the grounding wire contacts the user's hand.

12. An electrostatic generation device for an atomizer, comprising a housing (1), a water outlet pipe (3), a fan (34) that blows air towards the water outlet pipe (3), a motor (45) that drives the fan (34) to rotate, a water tank (33), an electrostatic generator (51), and a power supply (46) that supplies power to the motor (45), **characterized in that**, a guide tube (37) is fixedly installed inside the water outlet pipe (3) for directing water away from the fan (34), the end of the guide tube (37) away from the fan (34) is provided with an air outlet (49), and a water outlet (52); one pole of the electrostatic generator (51) contacts the liquid to charge the liquid, and the manner of contact with the liquid is at least one of the following:
(1) one pole of the electrostatic generator is connected inside the water tank, contacting and charging the liquid;
(2) one pole of the electrostatic generator is connected at the water outlet, contacting and charging the liquid;
(3) one pole of the electrostatic generator is connected inside the pipe between the water tank and the water outlet, contacting and charging the liquid.

13. An electrostatic generation device for an atomizer, comprising a housing (1), a water outlet pipe (3), a fan (34) that blows air towards the water outlet pipe (3), a motor (45) that drives the fan (34) to rotate, a water tank (33), an electrostatic generator (51), and a power supply (46) that supplies power to the motor (45), **characterized in that**, a guide tube (37) is fixedly installed inside the water outlet pipe (3) for directing water away from the fan (34), the end of the guide tube (37) away from the fan (34) is provided with an air outlet (49), and a water outlet (52); the first electrode of the electrostatic generator (51) contacts the liquid, the second electrode does not contact the liquid, and the connection location of the second electrode is at least one of the following:
(1) the second electrode of the electrostatic generator is connected at the handle, contacting the user;
(2) the second electrode of the electrostatic generator is connected on a sharp needle in front of the water outlet;
(3) the second electrode of the electrostatic generator is connected on an electrostatic ring outside the water outlet.
